Europäisches Patentamt

European Patent Office

Office européen des brevets

(11) Numéro de publication: **0 394 108**
**A1**

## (12) DEMANDE DE BREVET EUROPEEN

(21) Numéro de dépôt: 90401011.3

(22) Date de dépôt: **12.04.90**

(51) Int. Cl.5: **G01N 23/12, G01N 33/20, G01N 27/27**

(30) Priorité: **18.04.89 FR 8905126**

(43) Date de publication de la demande:
**24.10.90 Bulletin 90/43**

(84) Etats contractants désignés:
**DE GB**

(71) Demandeur: **COMMISSARIAT A L'ENERGIE ATOMIQUE**
**31/33, rue de la Fédération**
**F-75015 Paris(FR)**

(72) Inventeur: **Pelletier, Thierry**
**20, rue R. Aron**
**F-21240 Talant(FR)**
Inventeur: **Gontier, Jacques**
**Les Crais - Bt D.**
**F-21380 Asnières Les Dijon(FR)**

(74) Mandataire: **Mongrédien, André et al**
**c/o BREVATOME 25, rue de Ponthieu**
**F-75008 Paris(FR)**

(54) **Installation de mesures en continu et en temps reel, de masses de métaux en solution acide, et de l'acidité de cette solution.**

(57) L'invention concerne une installation de mesures en continu et en temps réel, de masses de métaux en solution acide, et de l'acidité de cette solution.

Cette installation comporte une conduite (1) de circulation de cette solution, des moyens (2) traversés par la solution, pour mesurer l'acidité de celle-ci et fournissant un signal représentatif de la valeur de cette acidité, un débitmètre (4) relié à la conduite (1) et traversé par la solution et fournissant un signal représentatif de la valeur du débit de cette solution, une cellule (6) de circulation de la solution traversée par la solution, un spectromètre (7) agissant sur la cellule de circulation (6) et fournissant un signal représentatif de l'intensité des pics d'énergie correspondant respectivement aux valeurs des concentrations des métaux dans la solution, et un ordinateur (9) relié aux moyens de mesure d'acidité, au spectromètre et au débitmètre ; cet ordinateur est aussi relié à une mémoire (9) dans laquelle est enregistré un programme de calcul des masses respectives desdits métaux, à partir des valeurs des concentrations respectives et des valeurs de l'acidité et du débit.

FIG. 1

# INSTALLATION DE MESURES EN CONTINU ET EN TEMPS REEL, DE MASSES DE METAUX EN SOLUTION ACIDE, ET DE L'ACIDITE DE CETTE SOLUTION

La présente invention concerne une installation de mesures en continu et en temps réel de masses de métaux en solution acide et de l'acidité de cette solution. Elle s'applique plus particulièrement aux mesures en continu et en temps réel, de masses de métaux en solution d'acide nitrique, et notamment de masses de Plutonium, d'Uranium et d'Américium provenant d'usines de retraitement de déchets nucléaires et contenus dans cette solution d'acide nitrique.

Il n'existe pas actuellement d'installation permettant de mesurer en continu et en temps réel, des masses métalliques en solution acide et notamment de mesurer des masses d'Uranium, de Plutonium et d'Américium contenus dans une solution d'acide nitrique provenant d'une usine de retraitement de déchets nucléaires.

La seule méthode connue pour effectuer de telles mesures de masses ne permet pas d'obtenir des résultats en continu et en temps réel. Elle ne met pas en oeuvre un ensemble autonome pouvant être qualifié "d'installation de mesure". En effet, selon cette méthode connue, on prélève un faible échantillon de la solution acide contenant des masses métalliques et on effectue des dosages permettant de déterminer les valeurs de ces masses métalliques en solution.

Cette méthode présente de nombreux inconvénients : les mesures des masses effectuées par dosage sont réalisées à partir d'échantillons de la solution et non à partir de cette solution en totalité. Ces mesures ne sont donc pas effectuées en continu et en temps réel, elles sont très longues à mettre en oeuvre (plusieurs heures) et elles ne sont valables que pour un faible échantillon de la solution. De plus, notamment pour les mesures de masses d'uranium, de plutonium et d'américium, dans une solution d'acide nitrique, ces opérations de dosage doivent être effectuées manuellement, à l'intérieur d'une boîte à gants ; ces manipulations peuvent être dangereuses.

L'invention a pour but de remédier à ces inconvénients et notamment de réaliser une installation de mesures agissant en continu et en temps réel fournissant rapidement des résultats de mesures, concernant l'ensemble de la solution et sans nécessiter de manipulations longues et dangereuses.

Ces buts sont atteints en faisant circuler l'ensemble de la solution dans l'installation et en effectuant des mesures de débit de la solution, d'acidité, et de concentrations des métaux dans cette solution en circulation.

L'invention a pour objet une installation de mesure en continu et en temps réel de masses de métaux contenus en solution acide et de l'acidité de cette solution, caractérisée en ce qu'elle comporte une conduite de circulation de cette solution, des moyens reliés à la conduite et traversés par la solution, pour mesurer l'acidité de cette solution, une sortie de ces moyens de mesure d'acidité fournissant un signal représentatif de la valeur de cette acidité, un débitmètre relié à la conduite et traversé par la solution, pour fournir sur une sortie un signal représentatif de la valeur du débit de cette solution, une cellule de circulation de la solution, reliée à la conduite et traversée par la solution, un spectromètre agissant sur la cellule de circulation pour fournir sur une sortie un signal représentatif de l'intensité des pics d'énergie correspondant respectivement aux valeurs des concentrations des métaux dans la solution, et un ordinateur relié aux sorties des moyens de mesure d'acidité, du spectromètre et du débitmètre, cet ordinateur étant aussi relié à une mémoire dans laquelle est enregistré un programme de calcul des masses respectives desdits métaux, à partir des valeurs desdites concentrations respectives, et des valeurs de l'acidité et du débit.

Selon une autre caractéristique de l'invention, ladite cellule de circulation comporte un conduit d'arrivée de la solution relié à ladite conduite de circulation et débouchant dans un réservoir de transit, et un conduit d'évacuation de la solution dudit réservoir de transit vers un récipient de stockage, ledit spectromètre comportant une source d'irradiation de la solution, un détecteur de pics d'énergie, ayant une sortie constituant ladite sortie du spectromètre, et un puits cylindrique étanche d'irradiation de ladite cellule de circulation au cours des mesures, disposé verticalement entre la source d'irradiation et le détecteur, pour irradier la solution dans le réservoir de transit, ce puits d'irradiation ayant un fond et une paroi latérale cylindique et, à une extrémité supérieure, un orifice d'introduction de ladite cellule à l'intérieur dudit puits.

Selon une autre caractéristique, ladite cellule de circulation est un cylindre creux étanche contenant ledit réservoir de transit, ce cylindre représentant une base supérieure de laquelle débouchent lesdits conduits d'arrivée et d'évacuation de la solution, une base inférieure à proximité de laquelle est situé ledit réservoir de transit, et une enveloppe cylindrique solidaire desdites bases, l'introduction de ladite cellule de circulation dans ledit puits d'irradiation faisant apparaître un volume entre ladite enveloppe cylindrique de la cellule et l'intérieur dudit puits, ce volume étant fermé hermétiquement au moins à proximité de l'orifice dudit puits d'irradiation, par des joints solidaires de ladite enveloppe.

Selon une autre caractéristique, la cellule de circulation comporte un volume de détection de fuites de solution, situé à l'intérieur dudit cylindre entre la base inférieure de ce cylindre et le réservoir de transit, l'installation comportant en outre des moyens de détection de fuite de solution dans le volume de détection, ces moyens de détection étant reliés à une alarme.

Selon une autre caractéristique, les moyens de détection de fuite de solution comportent deux électrodes situées dans le volume de détection, ces électrodes étant reliées à des moyens de déclenchement d'alarme, par des moyens de connexion traversant la base inférieure de la cellule de circulation et le fond dudit puits d'irradiation.

Selon une autre caractéristique, la cellule de circulation comporte, au-dessus du réservoir de transit, à l'intérieur dudit cylindre, un volume d'expansion destiné à contenir la solution en cas de fuite.

Selon une autre caractéristique, la cellule de circulation comporte en outre un réceptacle d'attente de la cellule de circulation, pour la mise en attente de celle-ci, lorsqu'aucune mesure n'est effectuée, ce réceptacle ayant la forme d'un vase-support étanche, cylindrique, ayant un fond, une paroi latérale cylindrique et une extrémité supérieure présentant un orifice d'introduction de la cellule de circulation, l'introduction de la cellule de circulation dans le vase-support faisant apparaître un volume entre l'enveloppe cylindrique de la cellule et la paroi du vase-support, ce réceptacle comportant des moyens de détection de fuite de solution dans le volume entre l'enveloppe de la cellule et la paroi du vase-support, ces moyens étant reliés à ladite alarme.

Selon une autre caractéristique, le débitmètre et les moyens de mesure d'acidité sont situés dans une boîte à gants, ladite conduite traversant cette boîte à gants de façon étanche, lesdits conduits d'arrivée et d'évacuation de la solution traversant cette boîte à gants par une fenêtre ayant un pourtour délimité par un cadre, ce cadre étant relié hermétiquement par une manche souple étanche, à la base supérieure de la cellule de circulation, lesdits conduits d'arrivée et d'évacuation étant situées dans ladite manche, ledit conduit d'évacuation débouchant dans un récipient situé dans la boîte à gants, ledit réceptacle d'attente étant solidaire de la boîte à gants.

Selon une autre caractéristique, le spectromètre, l'ordinateur, les moyens de déclenchement d'alarme, l'alarme et le puits d'irradiation, forment un ensemble déplaçable à proximité de différentes boîtes à gants, chaque boîte à gants ayant ses propres débitmètre, moyens de mesure d'acidité, cellule de circulation, réceptacle d'attente et manche souple.

Selon une autre caractéristique, l'installation est destinée à la mesure des masses de Plutonium, d'Uranium et d'Américium, en solution d'acide nitrique, et à la mesure d'acidité de cette solution.

Selon une autre caractéristique les moyens de mesure d'acidité comportent une enceinte ayant un orifice d'entrée et un orifice de sortie de la solution, reliés à ladite conduite de circulation pour que cette enceinte soit traversée par la solution, une électrode de référence, et une électrode de mesure, plongeant dans la solution, à l'intérieur de ladite enceinte, dans une direction perpendiculaire à celle de la circulation de solution, ces deux électrodes étant reliées à un appareil de mesure de tension ayant une sortie constituant ladite sortie qui fournit un signal représentatif de l'acidité de la solution.

Les caractéristiques et avantages de l'invention ressortiront mieux de la description qui va suivre, donnée en référence aux dessins annexés dans lesquels :

- la figure 1 représente schématiquement une installation de mesure conforme à l'invention,

- la figure 2 représente schématiquement et en coupe longitudinale, une cellule de circulation de la solution, appartenant à l'installation de l'invention,

- la figure 3 représente schématiquement et en coupe, des moyens de mesures d'acidité de la solution, appartenant à l'installation de l'invention,

- la figure 4 représente schématiquement et en coupe longitudinale une cellule de référence utilisée dans l'installation de l'invention.

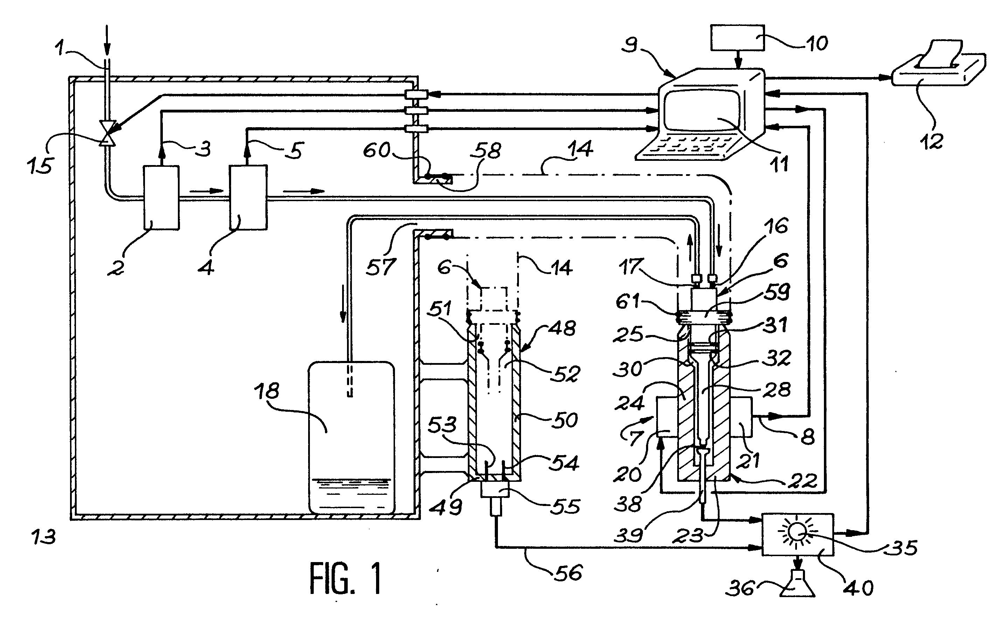

L'installation conforme à l'invention, représentée schématiquement sur la figure 1 comporte une conduite 1 de circulation de la solution acide contenant des masses de métaux, en solution qu'on souhaite déterminer. Elle comporte aussi des moyens 2 reliés à la conduite 1 et traversés par la solution, pour mesurer l'acidité de celle-ci. Une sortie 3 de ces moyens de mesure 2 fournit comme on le verra plus loin en détail, un signal représentatif de la valeur de cette acidité. La solution en circulation dans la conduite 1 provient d'une enceinte de stockage, non représentée.

Un débitmètre 4 est relié à la conduite 1, à la suite des moyens de mesure d'acidité 2. Ce débitmètre est traversé par la solution et fournit sur une sortie 5 un signal représentatif du débit de cette solution dans la conduite 1. Il est de type électromagnétique par exemple et ne sera pas décrit ici en détail.

L'installation comporte aussi une cellule 6 de circulation de la solution, cette cellule est reliée à la conduite 1, par exemple à la suite du débitmètre 4. Elle est traversée par la solution et sera décrite plus loin en détail.

Enfin, l'installation comporte un spectromètre 7 agissant sur la cellule de circulation 6 et fournissant sur une sortie 8, un signal représentatif de l'intensité des pics d'énergie correspondant respectivement aux valeurs des concentrations des différents métaux contenus dans la solution acide. Un ordinateur 9 est relié aux sorties 3, 5, 8 des moyens de mesure d'acidité 2, du débitmètre 4 et du spectromètre 7. Cette ordinateur est aussi relié à une mémoire 10 dans laquelle est enregistré un programme de calcul des masses respectives des métaux présent dans la solution acide ; ce calcul est effectué à partir des valeurs des concentrations respectives de ces métaux et des valeurs d'acidité et de débit de la solution. Ce calcul sera décrit plus loin en détail. L'ordinateur peut être muni d'un écran de visualisation 11 et être relié à une imprimante 12 permettant d'imprimer les résultats des mesures.

Dans un premier mode de réalisation, l'installation est utilisée pour des mesures de masses de métaux non radioactifs, on n'utilise alors pas de moyen de protection particulier ; dans un deuxième mode de réalisation l'installation est utilisée pour des mesures de masses de métaux radioactifs tels que le plutonium, l'uranium et l'américium ; l'installation comporte alors une boîte à gants 13 ainsi que différents éléments de protection tels qu'une manche souple étanche 14, qui seront décrits plus loin en détail.

On a aussi représenté sur la figure 1, une électrovanne 15 permettant de commander, comme on le verra plus loin en détail, la circulation ou l'arrêt de la circulation de la solution dans la conduite 1.

La cellule 6 de circulation de la solution comporte un conduit 16 d'arrivée de cette solution, et un conduit 17 d'évacuation de celle-ci. Le conduit d'arrivée 16 est relié à la conduite de circulation 1, par exemple à la suite du débitmètre 5. Le conduit d'évacuation 17 peut déboucher, par exemple, dans un bac de récupération 18 de la solution.

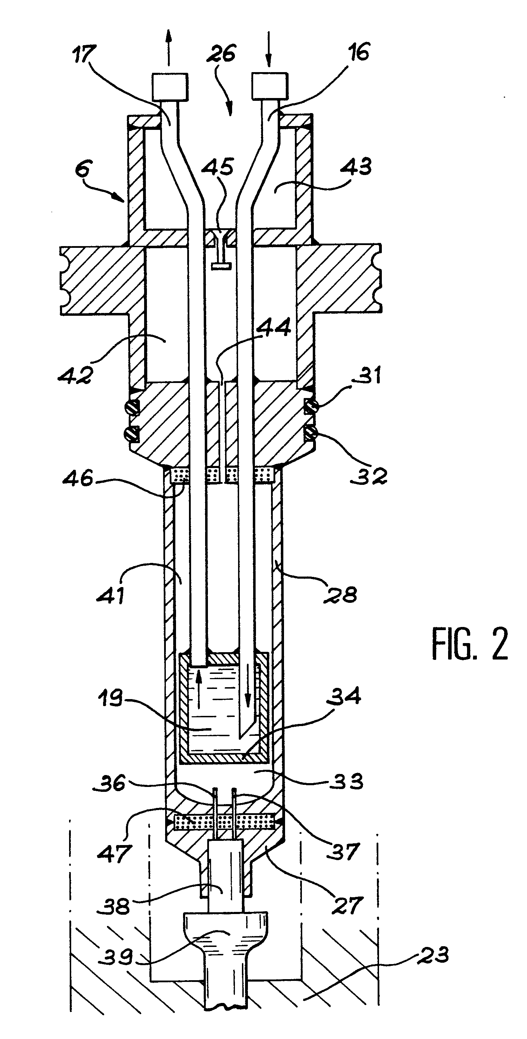

La structure de la cellule 6 de circulation de la solution sera mieux comprise à l'aide de la figure 2 qui représente cette cellule en coupe longitudinale. Le conduit 16 qui est relié à la conduite de circulation 1 et le conduit 17 qui est relié à une tubulure d'évacuation de la solution dans un récipient 18, débouchent dans un réservoir de transit 19. Cette figure sera décrite plus loin en détail.

En se référant à nouveau à la figure 1, le spectromètre 7 comporte une source 20, d'irradiation de la solution, et un détecteur 21 de pics d'énergie des radiations issus de la solution irradiée ; ces radiations sont par exemple des rayons X et/ou des rayons gamma . La source 20 et le détecteur sont connus dans l'état de la technique, et ne sont pas décrits ici en détail. Le détecteur 21 possède une sortie qui constitue la sortie 8 du spectromètre ; cette sortie fournit le signal représentatif des intensités des pics d'énergie correspondant respectivement aux différents métaux contenus dans la solution. Ces intensités sont proportionnelles aux concentrations respectives des différents métaux contenus dans la solution. L'installation comporte aussi un puits cylindrique étanche 22, l'irradiation de la cellule de circulation 6 ayant lieu au cours des mesures. Ce puits est disposé verticalement entre la source d'irradiation 20 et le détecteur 21, pour irradier la solution dans le réservoir de transit 19 (figure 2). Ce puits cylindrique d'irradiation est étanche ; il comporte un fond 23, une paroi latérale cylindrique 24 et, à une extrémité supérieure, un orifice 25 d'introduction de la cellule de circulation 6 à l'intérieur de ce puits.

Comme le montre la figure 2, la cellule de circulation 6 est un cylindre creux étanche contenant le réservoir de transit 19. Ce cylindre présente une base supérieure 26 de laquelle débouchent les conduits d'arrivée et d'évacuation 16, 17 de la solution, et une base inférieure 27, à proximité de laquelle est situé le réservoir de transit 19, à l'intérieur de la cellule. Les bases supérieure et inférieure sont réunies, de façon étanche, par une enveloppe cylindrique 28 ; cette enveloppe peut d'ailleurs être constituée par plusieurs enveloppes cylindriques de diamètres différents, réunies par des soudures non référencées sur cette figure.

En se référant à nouveau à la figure 1, on constate que l'introduction de la cellule de circulation 6 dans le puits d'irradiation 22, fait apparaître un volume 30 entre l'enveloppe 28 de la cellule 6 et l'intérieur de la paroi 24 du puits d'irradiation 22. Ce volume est fermé hermétiquement, au moins à proximité de l'orifice 25 du puits d'irradiation 22. Cette fermeture hermétique peut être réalisée par exemple au moyen de joints toriques 31, 32, s'engageant dans des gorges de l'enveloppe cylindrique 28 de la cellule 6, pour être rendus solidaires de celle-ci.

Comme le montre plus précisément la figure 2 la cellule d'irradiation 6 comporte aussi un volume 33 permettant de détecter une fuite éventuelle de la solution, normalement contenue dans le réservoir 19. Cette fuite peut aussi provenir des conduits 16 et 17 d'arrivée et d'évacuation. Ce volume est situé à l'intérieur du cylindre creux de la cellule 6, entre la base inférieure 27 de la cellule 6, et le fond 34 du réservoir de transit 19.

Des moyens de détection de fuite éventuelle de solution dans le volume de détection 33 sont reliés à une alarme visuelle 35 et/ou sonore 36. Ils sont constitués comme le montre la figure 2, par deux électrodes 36, 37 qui traversent par des passages isolants et étanches, la base inférieure 27 de la cellule 6 et qui débouchent dans le volume de détection 33. Lorsqu'il se produit une fuite de solution dans le volume 33, ces deux électrodes sont mises en court-circuit ; elles sont reliées à un connecteur mâle 38 qui coopère avec un connecteur femelle 39 traversant de façon étanche le fond 23 du puits d'irradiation 22. Les moyens de détection de fuite comportent aussi des moyens 40 de déclenchement d'alarme, reliés au connecteur femelle 39. Ces moyens de déclenchement d'alarme ne sont pas décrits ici en détail. Ils comportent notamment une source d'alimentation continue reliée aux électrodes, des moyens d'amplification d'un signal de court-circuit entre les électrodes, et des moyens de mise en forme de ce signal pour déclencher l'alarme, en cas de court-circuit entre les électrodes.

La cellule de circulation 6 présente, au-dessus du réservoir de transit 19, à l'intérieur du cylindre creux constituant cette cellule, un volume d'expansion constitué par exemple, dans le mode de réalisation représenté sur la figure 2, par des compartiments 41, 42 mis en communication par un conduit 44 et par un orifice muni d'une soupape 45. Ce volume d'expansion est destiné à contenir la solution en cas de fuite provenant des conduits 16, 17, ou du réservoir 19, au-dessus de ce réservoir.

Dans le mode de réalisation pour lequel l'installation est utilisée pour effectuer des mesures de masses de plutonium, d'uranium et d'américium contenus dans des solutions provenant d'usines de retraitement de déchets nucléaires, des disques de protection 46, 47 en plomb sont prévus à l'intérieur du cylindre creux constituant la cellule de circulation 6, ces disques sont respectivement situés vers les parties supérieure et inférieure de cette cellule, de part et d'autre du réservoir de transit 19. Ils ne sont pas prévus lorsque l'installation est utilisée pour effectuer des mesures concernant des produits non radioactifs, n'émettant pas de rayonnements dangereux.

L'installation comporte en outre, comme le montre la figure 1 un réceptacle 48 de mise en attente de la cellule de circulation 6 ; cette cellule est placée dans ce réceptacle, lorsqu'aucune mesure n'est effectuée. Ce réceptacle a la forme d'un vase-support étanche, cylindrique, ayant un fond 49 et une paroi latérale 50 de forme cylindrique. Ce vase-support 48 présente à une extrémité supérieure, un orifice 51 d'introduction de la cellule de circulation.

L'introduction de la cellule de circulation 6 dans le réceptacle 48 fait apparaître une volume 52 entre l'enveloppe cylindrique de la cellule 6 et la paroi latérale 50 du vase-support. Dans le mode de réalisation où l'installation est utilisée pour des mesures concernant des produits radioactifs, le réceptacle 48 comporte des moyens de détection d'une fuite éventuelle de la solution dans le volume 52, entre l'enveloppe de la cellule 6 et la paroi 48 du vase-support ; ces moyens de détection sont reliés à l'alarme visuelle 35 et/ou sonore 36. Ils sont constitués, comme précédemment, par deux électrodes 53, 54 reliées aux moyens 40 de déclenchement d'alarme, par l'intermédiaire d'un connecteur 55 et d'une liaison 56. En cas de fuite de solution dans le volume 52, un court-circuit s'établit entre les électrodes 53 et 54 et permet de déclencher l'alarme. Les moyens de déclenchement d'alarme 40 peuvent d'ailleurs être reliés à l'ordinateur 9 pour l'enregistrement de cette alarme et sa visualisation dans les résultats fournis par l'imprimante 12 et/ou l'écran 11. En cas d'alarme, l'ordinateur 9 arrête la circulation de la solution, en commandant la fermeture de l'électrovanne 15.

Dans le mode de réalisation pour lequel l'installation est utilisée pour des mesures concernant des produits radioactifs, comme indiqué plus haut, celle-ci comporte une boîte à gants 13, la conduite 1 traversant cette boîte à gants de façon étanche ; l'électrovanne 15 contenue dans cette boîte à gants est commandée par l'ordinateur 9, au moment où des mesures doivent être déclenchées. Les conduits 16, 17 d'arrivée et d'évacuation de la solution traversent la boîte à gants par une fenêtre 57 ayant un pourtour délimité par un cadre 58. Ce cadre est relié hermétiquement par la manche souple étanche 14 à une partie 59 de la base supérieure 26 de la cellule 6. Des colliers tels que 60, 61 permettent d'assurer l'étanchéité souhaitée. Les conduits 16, 17 d'arrivée et d'évacuation de la solution sont situés dans la manche 14, le conduit 17 débouchant comme indiqué plus haut dans le récipient 18 situé dans la boîte à gants 13. Dans ce mode de réalisation, le réceptacle 48 de la cellule de circulation est solidaire de la boîte à gants 13.

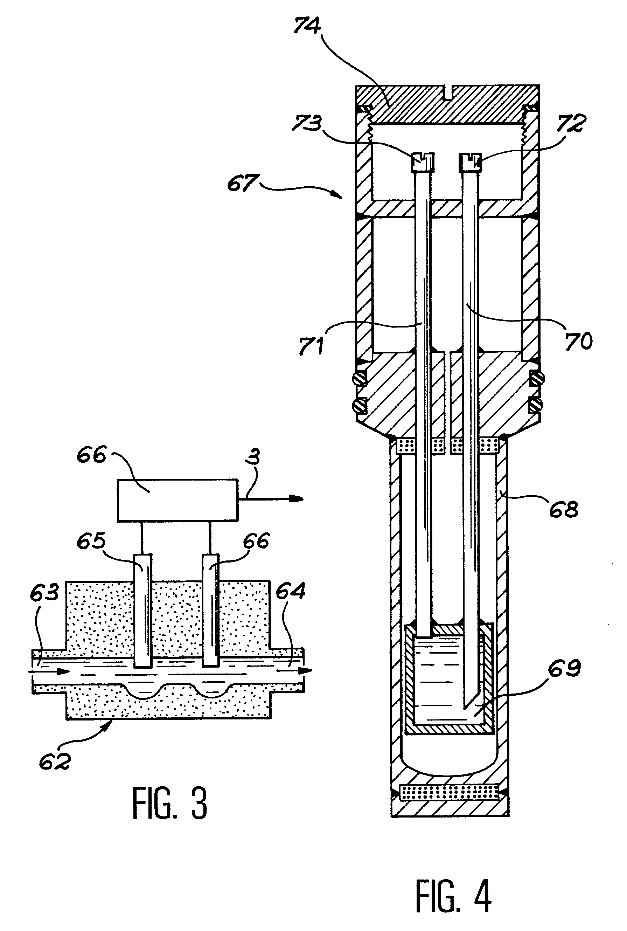

La figure 3 représente schématiquement et en coupe longitudinale, les moyens 2 de mesure de l'acidité de la solution. Ces moyens de mesure d'acidité comportent une enceinte 62 ayant un orifice d'entrée 63 et un orifice de sortie 64, reliés à la conduite de circulation 1 pour que cette enceinte soit traversée par la solution. Ces moyens comportent aussi une électrode de référence 65 et une électrode de mesure 66 plongeant dans la solution par leurs extrémités, à l'intérieur de cette enceinte, dans une direction perpendiculaire à celle de la circulation de la solution. Ces deux électrodes sont reliées à un appareil de mesure de tension 66, par exemple un millivoltmètre ayant une sortie 3 qui constitue la sortie fournissant le signal représentatif de l'acidité de la solution. Ce signal est fourni à l'ordinateur 9. Les électrodes 65, 66 sont de forme cylindrique et sont entourées d'un électrolyte (non représenté sur la figure), approprié à la mesure de l'acidité de la solution concernée. Ce sont par exemple des électrodes de type PHF100 commercialisées par la société TACUSSEL.

Pour des raisons qui seront expliquées plus loin en détail, l'installation comporte aussi, au moins une cellule de référence 67, représentée schématiquement et en coupe longitudinale sur la figure 4. Cette cellule comporte, comme la cellule de circulation un cylindre creux 68 à plusieurs compartiments ; elle est hermétiquement fermée et contient à sa partie inférieure, un réservoir 69 identique au réservoir de transit. Ce réservoir est rempli par une solution de référence, contenant des masses de valeurs connues, de métaux en solution. Cette cellule de référence permet l'étalonnage des mesures et elle est introduite dans le puits d'irradiation 22 de la figure 1 pour effectuer des mesures de référence. On a aussi représenté sur cette figure deux tubes 70, 71 identiques aux tubes d'arrivée d'évacuation de la solution, qui sont utilisés dans la cellule de circulation. L'un de ces tubes (70 par exemple) sert au remplissage du réservoir 69 par la solution de référence. Ces deux tubes, le réservoir 69, ainsi que toute la partie inférieure de la cellule de référence présentent des dimensions et une structure identiques à celles de la cellule de circulation, de manière à ne pas fausser les mesures de référence. Après remplissage du réservoir 69 par la solution de référence, les tubes 70, 71 sont respectivement fermés hermétiquement par des bouchons 72, 73, tandis que la partie supérieure du cylindre creux 68 est fermée hermétiquement par un bouchon 74.

Dans le mode de réalisation où l'installation est utilisée pour des mesures concernant des produits radioactifs, le spectromètre 7, le puits d'irradiation 22, l'ordinateur 9 ainsi que l'imprimante 12 et la mémoire 10 qui lui sont associées, les moyens de déclenchement d'alarme 40 et l'alarme (35 ou 36), forment un ensemble déplaçable à proximité de différentes boîtes à gants correspondant respectivement à différentes solutions contenant des produits radioactifs. Dans ce cas, chaque boîte à gants comporte ses propres débitmètres 4, moyens de mesure d'acidité 2, cellule de circulation 6, réceptacle d'attente 48 et manche souple 14. En effet, cet ensemble déplaçable comporte les moyens les plus coûteux de l'installation. Il est donc utile de pouvoir l'utiliser pour effectuer des mesures de différentes solutions parvenant respectivement à différentes boîtes à gants.

L'installation qui vient d'être décrite fonctionne de la manière suivante :
- on suppose par exemple que la solution est une solution d'acide nitrique contenant du Plutonium, de l'Uranium et de l'Américium en solution. A partir du clavier de l'ordinateur 9 et du programme contenu dans sa mémoire 10, on commande l'ouverture de l'électrovanne 15 pour assurer la circulation de la solution dans la conduite 1. Cette solution circule alors dans les moyens de mesure d'acicité 2, le débitmètre 4 et la cellule de circulation 6, la mise en marche de la source d'irradiation 20 et le début de la mesure ayant été commandés simultanément par l'ordinateur 9. Au cours de cette circulation la mémoire 10 de l'ordinateur enregistre périodiquement les valeurs de l'acidité, du débit et de la concentration des différents métaux dans la solution, fournis respectivement par les moyens de mesure d'acidité 2, le débitmètre 4 et le détecteur 21 du spectromètre.

La mesure de l'acidité de la solution permet de corriger dans les intensités des pics d'énergie fournis par le détecteur 22 du spectromètre, l'effet de la présence d'acide nitrique. On sait en effet que si l'on désigne par $I_2$ l'intensité des rayonnements issus d'une solution d'épaisseur x, cette solution étant irradiée par des rayonnements d'amplitude $I_1$, il est possible d'écrire $I_2 = I_1 e^{-\mu x}$.

Dans cette relation $\mu$ désigne le coefficient d'absorption de la matière traversée. Or, dans l'exemple considéré, cette matière contient de l'acide nitrique ainsi que des nitrates de Plutonium, d'Uranium et d'Américium.

Le coefficient d'absorption $\mu$ est en fait proportionnel à la somme des concentrations en Acide nitrique, en Plutonium, en Uranium et en Américium.

La concentration d'acide nitrique est connue grâce aux moyens de mesure d'acidité. Les mesures des intensités des pics d'énergie fournies par le détecteur, permettent alors de connaître les concentrations en Plutonium, en Uranium et Américium. La connaissance de ces concentrations et du débit de la solution permet d'en déduire les masses de ces différents métaux présents dans la solution.

Le calcul des concentrations est un calcul itératif permettant de corriger les valeurs des intensités des pics d'énergie fournis par le détecteur, ces pics étant souvent assortis de parasites et de traînées faussant les mesures. A cet effet, le programme de calcul enregistré dans la mémoire 10 de l'ordinateur permet, à partir des valeurs approchées des intensités des pics d'énergie, fournis par le détecteur et à partir des valeurs approchantes des intensités de pics d'énergie de solutions de référence mesurées à partir des cellules de référence mentionnées plus haut, d'effectuer des calculs itératifs de correction, permettant d'approcher au mieux les concentrations réelles des différents métaux dans la solution.

**Revendications**

1. Installation de mesures en continu et en temps réel, des masses de métaux en solution acide et de l'acidité de cette solution, caractérisée en ce qu'elle comporte une conduite (1) de circulation de cette solution, des moyens (2) reliés à la conduite (1) et traversés par la solution, pour mesurer l'acidité de cette solution, une sortie (3) de ces moyens de mesure d'acidité fournissant un signal représentatif de la valeur de cette acidité, un débitmètre (4) relié à la conduite (1) et traversé par la solution, pour fournir sur une sortie (5) un signal représentatif de la valeur du débit de cette solution, une cellule (6) de circulation de la solution, reliée à la conduite (1) et traversée par la solution, un spectromètre (7) agissant sur la cellule de circulation (6) pour fournir sur une sortie (8) un signal représentatif de l'intensité des pics d'énergie correspondant respectivement aux valeurs des concentrations des métaux dans la solution, et un ordinateur (9) relié aux sorties (3, 5, 8) des moyens de mesure d'acidité, du spectromètre et du débitmètre, cet ordinateur étant aussi relié à une mémoire (9) dans laquelle est enregistré un programme de calcul des masses respectives desdits métaux, à partir des valeurs desdites concentrations respectives, et des valeurs de l'acidité et du débit.

2. Installation selon la revendication 1, caractérisée en ce que ladite cellule de circulation (6) comporte un conduit (16) d'arrivée de la solution relié à ladite conduite de circulation (1) débouchant dans un réservoir (19) de transit, et un conduit (17) d'évacuation de la solution dudit réservoir de transit (19), vers un récipient de stockage (18), ledit spectromètre (7) comportant une source (20) d'irradiation de la solution, un détecteur (21) de pics d'énergie, ayant une sortie constituant ladite sortie (8) du spectromètre, et un puits cylindrique étanche (22) d'irradiation de ladite cellule de circulation (6) au cours des mesures, disposé verticalement entre la source d'irradiation (20) et le détecteur (21), pour irradier la solution dans le réservoir de transit (19), ce puits (22) d'irradiation ayant un fond (23) et une paroi (24) latérale cylindrique et, à une extrémité supérieure, un orifice (25) d'introduction de ladite cellule (6) à l'intérieur dudit puits.

3. Installation selon la revendication 2, caractérisée en ce que ladite cellule de circulation (6) est un cylindre creux étanche contenant ledit réservoir de transit (19), ce cylindre représentant une base supérieure (26) de laquelle débouchent lesdits conduits d'arrivée et d'évacuation (16, 17) de la solution, une base inférieure (27) à proximité de laquelle est situé ledit réservoir de transit (19), et une enveloppe cylindrique (28) solidaire desdites bases, l'introduction de ladite cellule de circulation (6) dans ledit puits d'irradiation (22) faisant apparaître un volume (30) entre ladite enveloppe cylindrique (28) de la cellule et l'intérieur dudit puits (22), ce volume (30) étant fermé hermétiquement au moins à proximité de l'orifice (25) dudit puits d'irradiation, par des joints (31, 32) solidaires de ladite enveloppe (28).

4. Installation selon la revendication 3, caractérisée en ce que la cellule de circulation (6) comporte un volume (33) de détection de fuites de solution, situé à l'intérieur dudit cylindre entre la base inférieure (27) de ce cylindre et le réservoir de transit (19), l'installation comportant en outre des moyens (36, 37, 40) de détection de fuite de solution dans le volume de détection, ces moyens de détection étant reliés à une alarme (35 et/ou 36).

5. Installation selon la revendication 4, caractérisée en ce que les moyens de détection de fuite de solution comportent deux électrodes (36, 37) situées dans le volume de détection (33), ces électrodes étant reliées à des moyens (40) de déclenchement d'alarme, par des moyens de connexion (38, 39) traversant la base inférieure (27) de la cellule de circulation (6) et le fond (23) dudit puits d'irradiation (22).

6. Installation selon la revendication 5, caractérisée en ce que ladite cellule de circulation (6) comporte, au-dessus du réservoir de transit (19), à l'intérieur dudit cylindre, un volume d'expansion (41, 42) destiné à contenir la solution en cas de fuite.

7. Installation selon la revendication 6, caractérisée en ce qu'elle comporte en outre un réceptacle (48) d'attente de la cellule de circulation (6), pour la mise en attente de celle-ci, lorsqu'aucune mesure n'est effectuée, ce réceptacle ayant la forme d'un vase-support étanche, cylindrique, ayant un fond (49), une paroi latérale cylindrique (50), et une extrémité supérieure présentant un orifice (51) d'introduction de la cellule de circulation (6), l'introduction de la cellule de circulation dans le vase-support faisant apparaître un volume (52) entre l'enveloppe cylindrique de la cellule (6) et la paroi (50) du vase-support, ce réceptacle comportant des moyens (53, 54, 40) de détection de fuite de solution dans le volume (52) entre l'enveloppe de la cellule et la paroi du vase-support, ces moyens étant reliés à ladite alarme (35 et/ou 36).

8. Installation selon la revendication 7, caractérisée en ce que le débitmètre (4) et les moyens de mesure d'acidité (2) sont situés dans une boîte à gants (13), ladite conduite (1) traversant cette boîte à gants de façon étanche, lesdits conduits (16, 17) d'arrivée et d'évacuation de la solution traversant cette boîte à gants (13) par une fenêtre (57) ayant un pourtour délimité par un cadre (58), ce cadre étant relié hermétiquement par une manche souple étanche (14), à la base supérieure (26) de la cellule de circulation (6), lesdits conduits d'arrivée et d'évacuation étant situés dans ladite manche, ledit conduit d'évacuation (17) débouchant dans un récipient (18) situé dans la boîte à gants (13), ledit réceptacle d'attente (48) étant solidaire de la boîte à gants.

9. Installation selon la revendication 8, caractérisée en ce que le spectromètre (7), le puits d'irradiation (22), l'ordinateur (9), les moyens de déclenchement d'alarme (40) et l'alarme, forment (35 et/ou 36) un ensemble déplaçable à proximité de différentes boîtes à gants, chaque boîte à gants ayant ses propres débitmètre, moyens de mesure d'acidité, cellule de circulation, réceptacle d'attente et manche souple.

10. Installation selon la revendication 8, caractérisée en ce qu'elle est destinée à la mesure des masses de Plutonium, d'Uranium et d'Amércicium, en solution dans l'acide nitrique, et à la mesure de l'acidité de cette solution.

11. Installation selon l'une quelconque des revendications 1 à 8, caracterisée en ce que les moyens (2) de mesure d'acidité comportent une enceinte (62) ayant un orifice (63) d'entrée et un orifice de sortie (64) de la solution, reliés à ladite conduite (1) de circulation pour que cette enceinte soit traversée par la solution, une électrode (65) de référence, et une électrode (66) de mesure, plongeant dans la solution, à l'intérieur de ladite enceinte, dans une direction perpendiculaire à celle de la circulation de solution, ces deux électrodes étant reliées à un appareil (66) de mesure de tension ayant une sortie (3) constituant ladite sortie qui fournit un signal représentatif de l'acidité de la solution.

12. Installation selon l'une quelconque des revendications 1 à 8, caractérisée en ce qu'elle comporte au moins une cellule de référence (67) comprenant un cylindre creux (68) hermétiquement fermé, contenant un réservoir (69) indentique au réservoir de transit (19), ce réservoir étant rempli par une solution de référence, contenant des masses de valeurs connues de métaux en solution, cette cellule de référence étant introduite dans le puits d'irradiation (22), pour effectuer des mesures de masses de référence.

FIG. 1

FIG. 2

FIG. 3

FIG. 4

Office européen
des brevets

**RAPPORT DE RECHERCHE EUROPEENNE**

Numero de la demande

EP 90 40 1011

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | PATENT ABSTRACTS OF JAPAN, vol. 100, no. 012 (P-683), 2 avril 1988; & JP-A-62 233 751 (TOSHIBA CORP.) 04-04-1986<br>--- | 1 | G 01 N 23/12<br>G 01 N 33/20<br>G 01 N 27/27 |
| A | FR-A-2 236 168 (AMF)<br>* Page 3, ligne 10 - page 4, ligne 16; page 7, lignes 23-29; page 9, lignes 14-39; page 12, ligne 30 - page 13, ligne 24; page 14, lignes 12-17; page 14, ligne 30 - page 15, ligne 35; page 16, lignes 33-36; figures 7,8 *<br>--- | 1 | |
| A | PATENT ABSTRACTS OF JAPAN, vol. 10, no. 206 (P478)[2262], 18 juillet 1986; & JP-A-61 47 551 (NIPPON KOKAN K.K.) 08-03-1986<br>--- | 1 | |
| A | US-A-4 512 853 (WRIGHT)<br>* Colonne 2, ligne 60 - colonne 3, ligne 63; résumé; figure 1 *<br>----- | 1 | |

**DOMAINES TECHNIQUES RECHERCHES (Int. Cl.5)**

G 01 N

Le présent rapport a été établi pour toutes les revendications

| Lieu de la recherche | Date d'achèvement de la recherche | Examinateur |
|---|---|---|
| LA HAYE | 16-07-1990 | BOSMA R.A.P. |